# EUROPEAN PATENT SPECIFICATION

(11) **EP 3 389 531 B1**
(45) Date of publication and mention of the grant of the patent: **06.05.2020**
(21) Application number: 16826596.5
(22) Date of filing: 23.12.2016
(51) Int. Cl.: A61B 18/14, A61B 17/29, A61B 17/32

(54) **AN ELECTROSURGICAL DEVICE**
ELEKTROCHIRURGISCHE VORRICHTUNG
DISPOSITIF ÉLECTROCHIRURGICAL

(30) Priority: 29.01.2016 US 201615010754
(43) Date of publication of application: 24.10.2018
(73) Proprietor: Gyrus ACMI, Inc., d.b.a. Olympus Surgical Technologies America, Southborough, MA 01772 (US)
(72) Inventor: TEIGEN, Lane, B., Maple Grove, MN 55311 (US)
(74) Representative: Manitz Finsterwald Patent- und Rechtsanwaltspartnerschaft mbB
(86) International application number: PCT/US2016/068522
(87) International publication number: WO 2017/131909

(56) References cited:
- WO-A2-2012/061722
- US-A1- 2006 293 656
- US-A1- 2012 316 473
- US-A1- 2015 209 573

## Description

### FIELD

The present invention relates to a medical device. More specifically, the present invention relates to an electrosurgical device.

### BACKGROUND

The statements in this section merely provide background information related to the present disclosure and may not constitute prior art.

Forceps may be utilized for laparoscopic surgery to control delicate movements inside a patient. The forceps typically include a gripping assembly to hold or grip an anatomical feature. The forceps also typically include a cutting assembly that employs electrical energy. Specifically, forceps may be used to seal vessels by delivering pulsed electrical energy to coagulate nearby tissue or may be used to cut tissue by delivering a higher level of energy to cut tissue. Among the literature that can pertain to this technology include the following patent document and published patent application: US 5,735,849 and US 2014/0135805.

In particular forceps, a physician squeezes a grip surface on a lever to control the amount of gripping force applied to the tissue of interest and then actuates a foot pedal to activate a generator, which supplies electrical energy to the forceps for cutting or coagulating the tissue. Such arrangements, however, may result in excessive gripping force being applied to the tissue, thereby tearing the tissue. A medical device according to the preamble of claim 1 is known from US2006293656.

Accordingly, there is a need for forceps that allows a physician to grip tissue with sufficient force without tearing the tissue while coagulating or cutting the tissue with the forceps.

### SUMMARY

The present invention provides a surgical device that has pressure sensing trigger and actuator.

Accordingly, pursuant to one aspect of the invention, there is contemplated a medical device including a handpiece, a lever with a grip surface attached to the handpiece, and a pressure sensor associated with the grip surface. When a user of the medical device applies a grip pressure to the lever, the lever moves to provide an input to a first function of the medical device, and concurrently the pressure sensor produces a pressure signal that is read by a control unit to control a second function.

The invention may be further characterized by one or any combination of the features described herein, such as, for example: the first function is a mechanical function; the medical device includes a pair of jaws with a first jaw and a second jaw, the second jaw being positioned adjacent to the first jaw, the pair of jaws being movable between an open position and a closed position; the mechanical function is an opening or a closing of the pair of jaws; the input to the mechanical function is a mechanical input to a mechanism that moves a tube over the pair of jaws to close the first jaw and the second jaw together so that they grip down on tissue; the input to the mechanical function is a mechanical input to a mechanisms that moves the pair of jaws beyond the end of the tube over to the first jaw and the second jaw; the first jaw includes a first electrode and the second jaw includes a second electrode, the first electrode and the second electrode being configured to deliver energy to tissue; the second function is an electrical function that provides energy to the pair of electrodes; the pressure signal determines the amount of energy provided to the pair of electrodes; an increased pressure signal is related to a continuous function; an increased pressure signal is related discretely from a first output to a second output; movement of the lever triggers an electrical switch to provide an electrical input; the electrical input activates an electrical subsystem; the electrical subsystem is a an indicator light or a debrider motor; the control unit activates the second function when the pressure signal is higher than a threshold pressure value; the threshold pressure value is related to a threshold valve that is greater than a force required to move the lever through its full range of motion; and the pressure sensor is a force sensing resistor that decreases in electrical resistance with increase in force applied to the pressure sensor.

Pursuant to another aspect of the present invention there is contemplated an electrosurgical device including a handpiece, an elongated tubular member with a proximal end and a distal end with the proximal end connected to the handpiece, a pair of jaws positioned at the distal end of the elongated tubular member, a lever with a grip surface attached to the handpiece, and a pressure sensor associated with the grip surface. The pair of jaws includes a first jaw with a first electrode and a second jaw with a second electrode. The second jaw is positioned adjacent to the first jaw, the pair of jaws is movable between an open position and a closed position, and the first electrode and the second electrode are configured to deliver energy to tissue. When a user of the medical device applies a grip pressure to the lever, the lever moves to open or close the pair of jaws, and concurrently the pressure sensor produces a pressure signal that is read by a control unit to control the amount of energy provided to the first jaw and the second jaw.

This aspect of the invention may be further characterized by one or any combination of the features described herein, such as, for example: movement of the lever triggers an electrical switch to provide an electrical input; the electrical input activates an indicator light; the amount of energy is sufficient to cut the tissue; the amount of energy is sufficient to coagulate the tissue; the control unit provides energy to the pair of jaws when the pressure signal is higher than a threshold pressure value; the threshold pressure value is related to a threshold force that is greater than a force required to move the lever through its full range of motion; and the pressure sensor is a force sensing resistor that decreases in electrical resistance with increase in force applied to the pressure sensor.

In yet another aspect of the invention, there is contemplated a method of treating a patient with a medical device with one or more of the following steps: inserting an elongated tubular member into the patient, the elongated tubular member having a proximal end connected to a handpiece and a distal end with a pair of jaws, the pair of jaws including a first jaw and a second jaw, the second jaw being positioned adjacent to the first jaw, the pair of jaws being movable between an open position and a closed position; positioning the pair of jaws at a desired anatomical region of the patient; and gripping a lever with a grip surface attached to the handpiece. Gripping the lever applies a grip pressure to the lever so that the lever moves to provide an input to a first function of the medical device, and concurrently a pressure sensor associated with the grip surface produces a pressure signal that is read by a control unit to control a second function.

This aspect of the invention may be further characterized by one or any combination of the features described herein, such as, for example: the first function is a mechanical function; the mechanical function is an opening and a closing of the pair of jaws; the first jaw includes a first electrode and the second jaw includes a second electrode, the first electrode and the second electrode being configured to deliver energy to tissue; the second function is an electrical function that provides energy to the pair of electrodes; the pressure signal determines the amount of energy provided to the pair of electrodes; the amount of energy is sufficient to cut the tissue; the amount of energy is sufficient to coagulate the tissue; the control unit only activates the second function when the pressure signal is higher than a threshold pressure value; the threshold pressure value is related to a threshold force that is greater than a force required to move the lever through its full range of motion; and the pressure sensor is a force sensing resistor that decreases in electrical resistance with increase in force applied to the pressure sensor.

Further features, advantages, and areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way. The components in the figures are not necessarily to scale, emphasis instead being placed upon illustrating the principles of the invention. In the drawings:
FIG. 1 is a side view of an electrosurgical device in accordance with the principles of the present invention;
FIG. 2 is an interior side view of the electrosurgical device shown in FIG. 1;
FIG. 3 is a close-up view of the region D shown in FIG. 2;
FIG. 4 is a close-up expanded view of a handle portion of the electrosurgical device shown in FIG. 1; and
FIG. 5 is a cross-sectional partial view taken along the line 5-5 in FIG. 2.

### DETAILED DESCRIPTION

The following description is merely exemplary in nature and is not intended to limit the present disclosure, application, or uses.

Referring now to the drawings, an electrosurgical or medical device embodying the principles of the present invention is illustrated therein and designated at 10. The electrosurgical device 10 is designed for use in percutaneous laparoscopic surgical procedures.

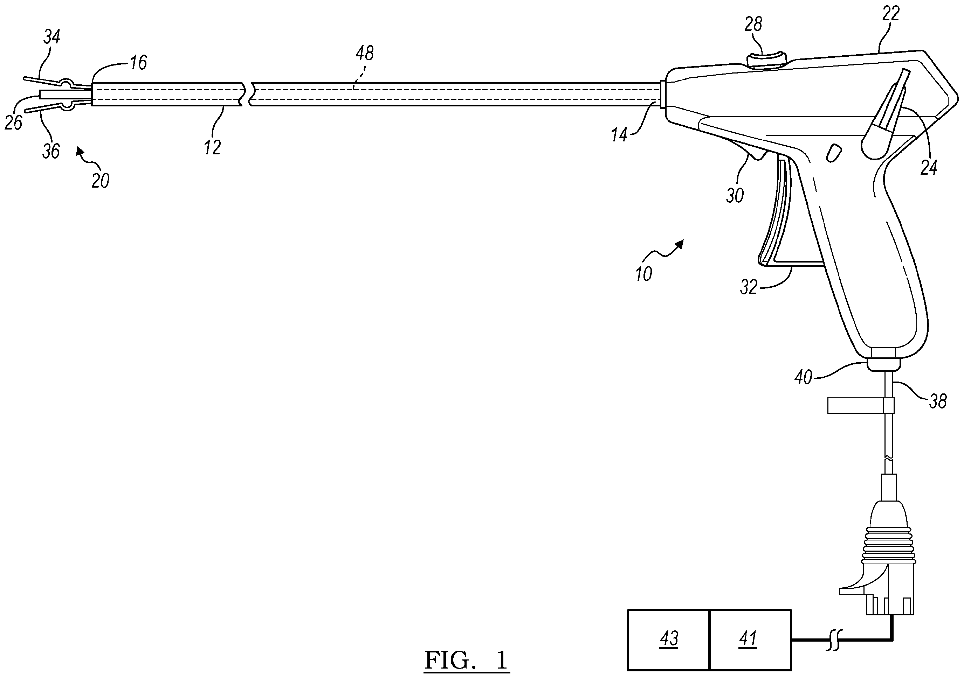

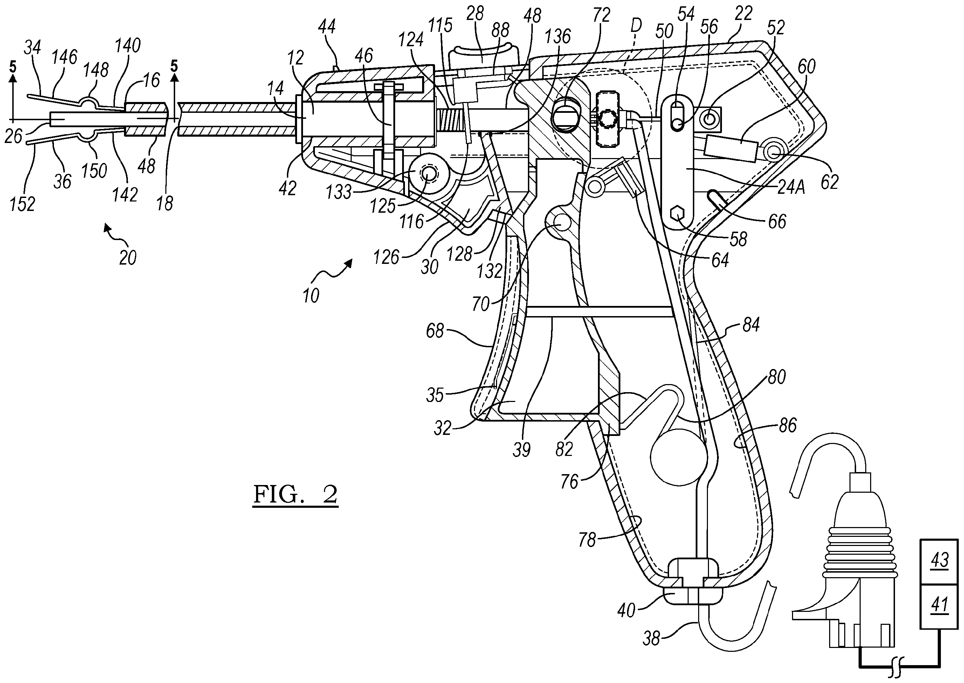

Referring specifically to FIGS. 1 and 2, the electrosurgical device 10 is a cutting forceps and includes an elongated tubular barrel member 12, which may be formed from the variety of materials, such as, for example, stainless steel. The outer tubular barrel member has a proximal end 14, a distal end 16 and a lumen 18 extending the entire length thereof.

A cutting and coagulating jaw arrangement 20 extends from the distal end 16, and a handle or handpiece 22 is located at the proximal end 14. The handle 22 includes a pivoting blade lever 24 for actuating the cutting blade 26 of the cutting and coagulating jaw arrangement 20. A thumb slide 28 is located on the top of the handle 22 for selecting either the bidirectional mode or the unidirectional mode of the cutting and coagulating forceps movement, as will be explained later. A trigger lock release 30, which allows bidirectional movement when the unidirectional mode is selected, is located adjacent a lever 32. Lever 32 is manipulated for actuating the coagulating jaws 34 and 36 of the cutting and coagulating jaw arrangement 20. Insulated electrical leads designated 38 extend from an electrical connector 40 on the handle 22 and are adapted to be connected to a source of RF power, such as, for example, a generator 41.

Turning further to FIG. 2, the interior of handle 22 is shown. The proximal end 14 of the barrel member 12 extends into a bore 42 formed in the distal end 44 of the handle 22. The bore 42 enlarges, forming an annular recess which receives a ring member 46. The ring member 46 is affixed to the barrel member 12 thus securing it to the handle 22.

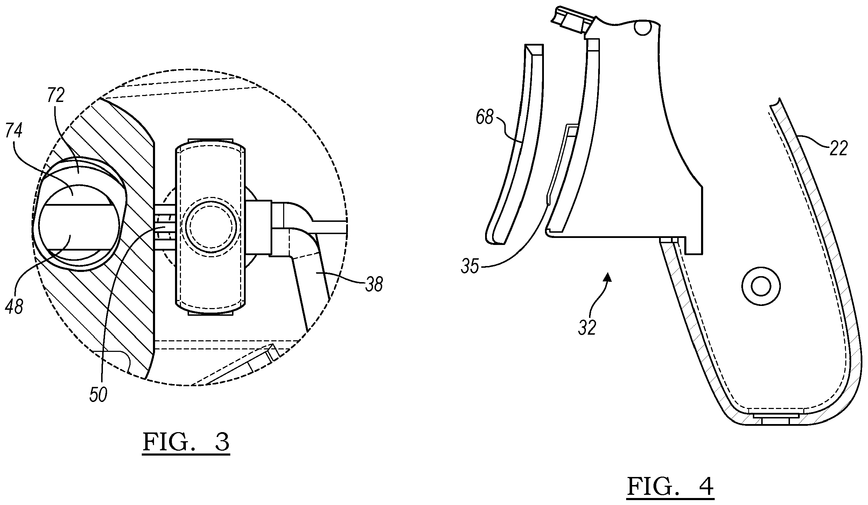

Projecting out from the proximal end of the barrel member 12 into the handle 22 interior is an inner tubular member 48 and a blade rod 50. The blade rod 50 terminates in a clevis arrangement 52. The pivoting blade lever 24 shown in FIG. 1 has an interior lever 24a as seen in FIG. 2. The lever 24a is coupled to the blade rod 50 (see also FIG. 3) through an elongate slot 54 in which a pin 56 is positioned. The blade rod 50, in turn, is connected to the cutting blade 26. The lever 24a is pivotally attached to the handle 22 at the pivot pin 58. The levers 24 and 24a can be integrally formed or coupled such that they move in unison. A helical tension spring 60 is fixed to a mid-point of the lever 24a. The spring 60 is also fixed to a boss formed on the rear interior surface 62 of the handle 22. Two stops are formed in the handle cavity for limiting the range of movement of the levers 24 and 24a and in turn the movement of the blade 26. Specifically, a stop 64 limits forward movement of the levers 24 and 24a, blade rod 50 and blade 26, and a stop 66 limits the rearward movement of lever 24a, blade rod 50 and blade 26.

As seen in FIG. 2, an opening is formed in the handle 22 through which a grip surface or finger engaging portion 68 of trigger or lever 32 extends. The lever 32 is pivotally secured to the handle interior through a pivot pin 70. A slot 72 is located in the upper portion of the lever 32. The inner tubular member 48 terminates at a clevis pin arrangement 74 (FIG. 3) which is coupled to the lever 32 through engagement with the slot 72. The lever 32 also includes an extension 76 which extends past the opening and contacts an interior surface 78 of the handle 22. A torsion or return spring 80 is located in handle 22 with one end 82 resting against the lever 32 and the other end 84 resting against a rear interior surface 86 of the handle 22. The return spring 80 acts as a biasing means for the lever 32 providing a return force to the lever.

The slide 28 for selecting either the unidirectional mode or bidirectional mode for moving the jaw arrangement is positioned in a slot 88 formed on the upper portion of handle 22. The slide 28 has a locking member 115 that receives an edge of a locking washer 116 as seen in FIG. 2. The locking washer 116 has a generally rectangular shape, and the inner tubular member 48 extends through an aperture of the locking washer 116. A helical compression spring 124 concentrically surrounds inner tubular member 48 and is positioned between the proximal end 14 of the barrel member 12 and the washer 116 and acts as a biasing means for the locking washer 116.

The locking washer 116 has two positions. In the first position or unlocked position, the locking washer is substantially perpendicular to the inner tubular member 48 such that the locking washer's aperture loosely surrounds the inner tubular member 48. In the second position or locked position, the locking washer 116 is tipped so that the edge defining the aperture frictionally engages the inner tubular member 48. The position of washer 116 is determined by the position of thumb slide 28. When thumb slide 28 is in the forward position, the locking washer 116 is in the locked position, and when the thumb slide 28 is in the rearward position, the locking washer 116 is in the unlocked position.

The lock release trigger 30 is located below the locking washer 116 within easy reach by a user's forefinger. The trigger 30 is pivotally secured to the handle through pivot pin 125 with a finger engaging portion 126 extending out through an opening in handle 22. The trigger 30 includes a stop 128 which extends past the opening on the handle interior and normally rests against an interior wall surface 132. A torsion spring 133 is secured at the pivot pin 125. The end of the torsion spring 133 rests against the interior wall 132 of handle 22. The other end of the torsion spring 133 rests against an arcuate surface formed on the trigger 30. An upper surface 136 of trigger 30 selectively contacts the locking washer 116.

The cutting and coagulating apparatus 20 includes a first jaw member 34 and a second jaw member 36 to perform the grasping, coagulating and cutting functions of the forceps. The jaws 34, 36 extend from the distal end 16 of the inner tubular member 48 and barrel member 12. Two pairs of conductive wire leads 140 and 142 and the cutting blade rod 50 extend through the entire length of the inner tubular member 48. The leads extend out of the handle 22 and together are identified as insulated electrical leads 38. A scalpel blade 26 or other cutting tool, such as a wire with a sharpened leading and trailing edge, is crimped on the distal end of the blade rod 50 and therefore is movable therewith.

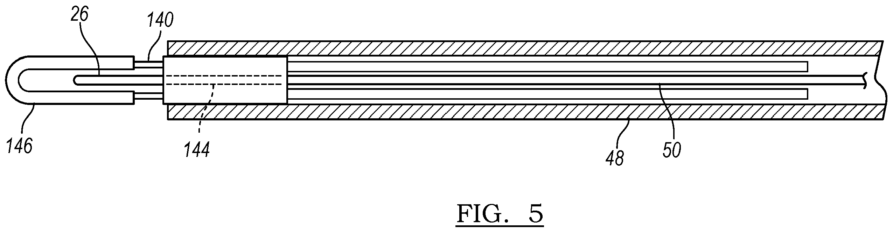

A guide sleeve support 144 is press fit in the distal end of the inner tubular member 48 as shown in FIG. 5. The guide sleeve support 144 contains longitudinal bores through which the leads 140 and 142 and the blade 26 and associated blade rod 50 extend. The first pair of conductive leads 140 form the first jaw 34 of the forceps. The wire pair 140 passes through the guide sleeve support 144 and extends from the distal end 16 of the tubular member in spaced apart parallel arrangement, forming a slot for receiving the cutting blade 26. The wires 140 form a generally U-shaped flattened electrode surface or first electrode 146 at their distal end with an arcuate configuration 148 just proximal of the flattened electrode surface of the first jaw 34.

The second conductive wire lead pair 142 forms the second jaw 36 of the coagulating forceps. The second conductive pair 142 extends, like the first pair 140, from the distal end 16 of the tubular member 12 in spaced apart parallel arrangement to each other forming a slot so that the cutting blade 26 can pass freely therebetween. Like the first conductive pair 140, the second pair 142 has an arcuate portion 150 prior to forming a generally U-shaped flat electrode surface or second electrode 152.

The arcuate configuration of the conductive wire pairs 140 and 142 accomplish the closing of the forceps. The guide sleeve support 144 is moved in the distal direction over the arcuate portions 148 and 150 of the wires 140 and 142 as he inner tubular member 48 is moved toward the distal end of the forceps. The arcuate portions 148 and 150 are squeezed together, closing the coagulating jaw pair 34 and 36 against one another or against tissue disposed therebetween. The guide sleeve support 144 is configured to move longitudinally inside the outer tubular member 12 and over the leads 140 and 142 by effectuating longitudinal displacement of the inner tubular member 48 via the lever 32.

The forceps 10 is intended to be used in conjunction with a cannula, however it is not limited to such use. As such, the forceps 10 tubular member 12 is inserted through the lumen of a cannula extending into the patient. The surgeon views the surgical site with a laparoscope. The jaws 34 and 36 are positioned about the tissue to be cut and coagulated. The surgeon selects either the unidirectional mode or the bidirectional mode for actuating the jaws.

In the unidirectional mode, the jaws 34 and 36 can only move towards each other to close on the tissue to be grasped, cut and coagulated. If pressure on the lever 32 is released, the jaws 34 and 36 remain in the position they were in when the pressure was released. Thus, if the jaws are partially closed, the jaws will remain in the partially closed position. In the bidirectional mode, the jaws can move in both directions in order to close or open. Thus, when the pressure on the lever 32 is released, the jaws open under influence of the return spring 80.

The unidirectional mode is chosen by moving the thumb slide 28 towards the distal end of handle 22. This causes the locking washer 116 to tip towards the distal end of the forceps 10. The lever 32 is depressed to actuate the jaws 34 and 36. As lever 32 pivots, it moves the inner tubular member 48 towards the distal end of forceps 10. This movement causes the guide sleeve support 144 to reach the arcuate portions 148 and 150 of the wire conductors 140 and 142 causing them to be drawn together thereby closing jaws 34 and 36 onto the tissue.

As the surgeon closes the jaws 34 and 36, he or she may intermittently stop applying pressure to the lever 32. The jaws will remain in their position when the surgeon stops applying pressure to the lever 32 because the frictional forces between the tipped washer 116 and the inner tubular member 48 prevent movement of the tubular member 48 in the rearward direction. The surgeon may then reapply pressure to lever 32 for closing the jaws 34 and 36 further. Each time the surgeon stops compressing the trigger 32, the jaws remain in the position where the finger pressure is relieved. This allows the surgeon to incrementally close the jaws and control the amount of compression applied to the tissue structures involved.

If the surgeon determines the jaws 34 and 36 have been closed too much, the lock release trigger 30 may be used to temporarily release the lock washer 116. The surgeon depresses the release trigger 30, thus causing the locking washer 116 to return to its perpendicular position relative to the inner tubular member 48. Because the inner tubular member 48 is no longer held in position with friction, it will move and open the jaws 34 and 36 by the return spring 80. When the lock release trigger 30 is released, the locking washer 116 resumes its tipped position.

In the event the surgeon wishes to have bidirectional movement, the locking thumb slide 28 is moved to its rearward position, such that the locking washer 116 is perpendicular to the inner tubular member 48. This allows the inner tubular member 48 to reciprocate back and forth without encountering any frictional forces from the edges of the locking washer 116 defining its aperture. The jaws 34 and 36 will close when pressure is applied to lever 32 causing the guide sleeve support 144 to move over the arcuate portion 148 and 150 of the jaws 34 and 36. The jaws 34 and 36 will open when pressure on the lever 32 is released.

Referring to FIG. 4, the lever 32 includes a sensor 35 enclosed within in the grip surface 68. The sensor 35 is electrically connected to the electrical leads 38 with an additional lead 39 (shown in FIGS. 2 and 3). The sensor 35 can be, for example, a force sensing resistor, manufactured by Interlink Electronics, located in Westlake Village, CA, that decreases in electrical resistance as the force applied to the sensor 35 increases.

When the surgeon grips the grip surface 68 of the lever 32, the sensor 35 produces a pressure signal that is read by a control unit or controller 43, which, in turn, controls the amount of electrical energy the generator 41 supplies to the electrode surfaces 146 and 152. The increased pressure signal can be related to a continuous function, for example, power. Some generators have a cap on the voltage (V) and current (A), such as AC, delivered to the tissue, since the amount of power (V×A) can be quite high. Accordingly, to reduce the amount of power and therefore heat delivered to the tissue, a duty cycle can be applied to the maximum current so that there is a continuous function of the amount of power delivered to the tissue by alternating the duty cycle of the AC. Hence, the amplitude of the voltage can be a continuous function as well.

Alternatively, the increased pressure signal is related discretely from a first output to a second output, such as, for example, power as described above. In some arrangements, the controller 43 activates the generator 41 when the pressure signal from the sensor 35 exceeds a threshold pressure value. The threshold pressure value can be related to a threshold value that is greater than a force required to move lever 32 through its full range of motion. In certain arrangements, movement of the lever 32 triggers an electrical switch to provide an electrical input. The electrical switch may be the sensor 35 or it may be a separate switch. The electrical input may activate an indicator light. The electrical input may activate an electromechanical subsystem such as, for example, a debrider device to remove dead or contaminated tissue and foreign matter from a wound. Specifically, the lever 32 can be employed in conjunction with the debrider device in which movement of the lever 32 turns on the spinning debrider motor and the sensor 35 is employed to activate a bipolar electrosurgical circuit.

Accordingly, when the surgeon has closed the jaws 34 and 36 on the tissue with a desired gripping force and is ready to cut and coagulate the tissue, the controller 43 receives a signal from the sensor 35 to activate the generator 41. The generator 41 causes a current path to be developed between the electrode surfaces 146 and 152 on the jaws to heat and coagulate or electrosurgically cut the tissue pinched between the jaw electrodes. Further, the tissue can also be cut by pivoting the blade lever 24 of the handle 22 towards the distal end of handle 22. This causes the blade rod 50 to be longitudinally moved towards the distal end, causing the blade 26 to extend from the distal end 16 of the tubular member 12 between the conductor pairs and within the blade receiving slot. Once the tissue is cut, the blade 26 is retracted by removing pressure from the lever 32. The forceps jaws 34 and 36 may be released by temporarily releasing the locking mechanism through the lock release trigger 30 or by moving the slide 28 to the unlocked or rearward position, depending upon whether the unidirectional or bidirectional mode has been selected.

## Claims

1. A medical device (10) comprising:
a handpiece (22);
a lever (32) attached to the handpiece (24), the lever having a grip surface (68); and
a pressure sensor (35) associated with the grip surface (68), and
wherein the medical device is further configured, such that when a user of the medical device (10) applies a grip pressure to the lever (32), the lever (32) moves to provide an input to a first function of the medical device (10), and concurrently the pressure sensor (35) produces a pressure signal that is read by a control unit (43) to control a second function, wherein the first function is in particular a mechanical function, **characterised in that** said pressure sensor (35) is enclosed within the grip surface (68).

2. The medical device (10) of claim 1 further comprising a pair of jaws (34, 36) with a first jaw (34) and a second jaw (36), the second jaw (36) being positioned adjacent to the first jaw (34), the pair of jaws (34, 36) being movable between an open position and a closed position, wherein the mechanical function is an opening or a closing of the pair of jaws (34, 36).

3. The medical device (10) of claim 2 wherein the input to the mechanical function is one of:
a mechanical input to a mechanism that moves a tube (48) over the pair of jaws (34, 36) to close the first jaw (34) and the second jaw (36) together so that they grip down on tissue, and
a mechanical input to a mechanism that moves the pair of jaws (34, 36) beyond the end of the tube (48) to open the first jaw (34) and the second jaw (36).

4. The medical device (10) of claim 2 wherein the first jaw (34) includes a first electrode (146) and the second jaw (36) includes a second electrode (152), the first electrode (146) and the second electrode (152) being configured to deliver energy to tissue.

5. The medical device (10) of claim 4 wherein the second function is an electrical function that provides energy to the pair of electrodes (146, 152).

6. The medical device (10) of claim 5 wherein the pressure signal determines the amount of energy provided to the pair of electrodes (146, 152), and wherein an increased pressure signal is related to a continuous function.

7. The medical device (10) of claim 5 wherein the pressure signal determines the amount of energy provided to the pair of electrodes (146, 152), wherein an increased pressure signal is related discretely from a first power output to a second power output.

8. The medical device (10) of any of the preceding claims wherein movement of the lever (32) triggers an electrical switch to provide an electrical input.

9. The medical device (10) of claim 8 wherein the electrical input activates an electrical subsystem which is in particular one of an indicator light and a debrider motor.

10. The medical device (10) of any of the preceding claims wherein the control unit (43) activates the second function when the pressure signal is higher than a threshold pressure value.

11. The medical device (10) of claim 10 wherein the threshold pressure value is related to a threshold value that is greater than a force required to move the lever (32) through its full range of motion.

12. The medical device (10) of any of the preceding claims wherein the pressure sensor (35) is a force sensing resistor that decreases in electrical resistance with increase in force applied to the pressure sensor (35).

13. The medical device (10) of claim 1 further comprising:
an elongated tubular member (12) with a proximal end (14) and a distal end (16), the proximal end (14) being connected to the handpiece (22);
a pair of jaws (34, 36) positioned at the distal end (14) of the elongated tubular member (12), the pair of jaws (34, 36) including a first jaw (34) with a first electrode (146) and a second jaw (36) with a second electrode (152), the second jaw (36) being positioned adjacent to the first jaw (34), the pair of jaws (34, 36) being movable between an open position and a closed position, the first electrode (146) and the second electrode (152) being configured to deliver energy to tissue;
wherein the first function is to open or close the pair of jaws (34, 36), and the second function is an amount of energy provided to the first electrode (146) and the second electrode (152), wherein the amount of energy is in particular sufficient to coagulate the tissue.

14. The medical device (10) of claim 13 wherein the control unit (43) provides energy to the pair of jaws (34, 36) when the pressure signal is higher than a threshold pressure value.

## Patentansprüche

1. Medizinische Vorrichtung (10), umfassend:
ein Handstück (22);
einen Hebel (32), der an dem Handstück (24) befestigt ist, wobei der Hebel eine Grifffläche (68) aufweist; und
einen Drucksensor (35), der der Grifffläche (68) zugeordnet ist, und
wobei die medizinische Vorrichtung ferner derart konfiguriert ist, dass, wenn ein Nutzer der medizinischen Vorrichtung (10) einen Griffdruck auf den Hebel (32) ausübt, der Hebel (32) sich bewegt, um einen Eingang zu einer ersten Funktion der medizinischen Vorrichtung (10) bereitzustellen, und gleichzeitig der Drucksensor (35) ein Drucksignal erzeugt, das von einer Steuereinheit (43) gelesen wird, um eine zweite Funktion zu steuern, wobei die erste Funktion insbesondere eine mechanische Funktion ist, **dadurch gekennzeichnet, dass** der Drucksensor (35) in der Grifffläche (68) eingeschlossen ist.

2. Medizinische Vorrichtung (10) nach Anspruch 1, ferner mit einem Paar von Klauen (34, 36) mit einer ersten Klaue (34) und einer zweiten Klaue (36), wobei die zweite Klaue (36) benachbart der ersten Klaue (34) positioniert ist, wobei das Paar von Klauen (34, 36) zwischen einer offenen Position und einer geschlossenen Position bewegbar ist, wobei die mechanische Funktion ein Öffnen oder Schließen des Paares von Klauen (34, 36) ist.

3. Medizinische Vorrichtung (10) nach Anspruch 2, wobei der Eingang zu der mechanischen Funktion einer ist aus:
einem mechanischen Eingang zu einem Mechanismus, der ein Rohr (48) über das Paar von Klauen (34, 36) bewegt, um die erste Klaue (34) und die zweite Klaue (36) zusammen zu schließen, so dass sie in Richtung Gewebe greifen, und
einen mechanischen Eingang zu einem Mechanismus, der das Paar von Klauen (34, 36) über das Ende des Rohres (48) hinausbewegt, um die erste Klaue (34) und die zweite Klaue (36) zu öffnen.

4. Medizinische Vorrichtung (10) nach Anspruch 2, wobei die erste Klaue (34) eine erste Elektrode (146) aufweist und die zweite Klaue (36) eine zweite Elektrode (152) aufweist, wobei die erste Elektrode (146) und die zweite Elektrode (152) zur Lieferung von Energie an Gewebe konfiguriert sind.

5. Medizinische Vorrichtung (10) nach Anspruch 4, wobei die zweite Funktion eine elektrische Funktion ist, die Energie an das Paar von Elektroden (146, 152) liefert.

6. Medizinische Vorrichtung (10) nach Anspruch 5, wobei das Drucksignal die Energiemenge bestimmt, die an das Paar von Elektroden (146, 152) geliefert wird, wobei ein erhöhtes Drucksignal einer kontinuierlichen Funktion zugeordnet ist.

7. Medizinische Vorrichtung (10) nach Anspruch 5, wobei das Drucksignal die Energiemenge, die an das Paar von Elektroden (146, 152) geliefert wird, bestimmt, wobei ein erhöhtes Drucksignal diskret von einem ersten Leistungsausgang zu einem zweiten Leistungsausgang zugeordnet ist.

8. Medizinische Vorrichtung (10) nach einem der vorhergehenden Ansprüche, wobei eine Bewegung des Hebels (32) einen elektrischen Schalter auslöst, um einen elektrischen Eingang bereitzustellen.

9. Medizinische Vorrichtung (10) nach Anspruch 8, wobei der elektrische Eingang ein elektrisches Teilsystem aktiviert, das insbesondere ein Anzeigelicht oder ein Debrider-Motor ist.

10. Medizinische Vorrichtung (10) nach einem der vorhergehenden Ansprüche, wobei die Steuereinheit (43) die zweite Funktion aktiviert, wenn das Drucksignal höher als ein Schwellendruckwert ist.

11. Medizinische Vorrichtung (10) nach Anspruch 10, wobei der Schwellendruckwert einem Schwellenwert zugeordnet ist, der größer als eine Kraft ist, die erforderlich ist, um den Hebel (32) über seinen vollständigen Bewegungsbereich zu bewegen.

12. Medizinische Vorrichtung (10) nach einem der vorhergehenden Ansprüche, wobei der Drucksensor (35) ein Krafterfassungswiderstand ist, der einen elektrischen Widerstand mit Zunahme einer auf den Drucksensor (35) ausgeübten Kraft verringert.

13. Medizinische Vorrichtung (10) nach Anspruch 1, ferner umfassend:
ein längliches rohrförmiges Element (12) mit einem proximalen Ende (14) und einem distalen Ende (16), wobei das proximale Ende (14) mit dem Handstück (22) verbunden ist;
ein Paar von Klauen (34, 36), die an dem distalen Ende (14) des länglichen rohrförmigen Elements (12) positioniert sind, wobei das Paar von Klauen (34, 36) eine erste Klaue (34) mit einer ersten Elektrode (146) und eine zweite Klaue (36) mit einer zweiten Elektrode (152) aufweist, wobei die zweite Klaue (36) benachbart der ersten Klaue (34) positioniert ist, wobei das Paar von Klauen (34, 36) zwischen einer offenen Position und einer geschlossenen Position bewegbar ist, wobei die erste Elektrode (146) und die zweite Elektrode (152) konfiguriert sind, um Energie an Gewebe zu liefern;
wobei die erste Funktion darin besteht, das Paar von Klauen (34, 36) zu öffnen oder zu schließen, und die zweite Funktion eine Energiemenge ist, die an die erste Elektrode (146) und die zweite Elektrode (152) geliefert wird, wobei die Energiemenge insbesondere ausreichend ist, das Gewebe zu koagulieren.

14. Medizinische Vorrichtung (10) nach Anspruch 13, wobei die Steuereinheit (43) Energie an das Paar von Klauen (34, 36) liefert, wenn das Drucksignal höher als ein Schwellendruckwert ist.

## Revendications

1. Dispositif médical (10) comprenant :
une pièce à main (22) ;
un levier (32) fixé à la pièce à main (24), le levier présentant une surface de préhension (68) ; et
un capteur de pression (35) associé à la surface de préhension (68), et
dans lequel le dispositif médical est en outre configuré de telle sorte que lorsqu'un utilisateur du dispositif médical (10) applique une pression de préhension sur le levier (32), le levier (32) se déplace pour fournir une entrée à une première fonction du dispositif médical (10) et simultanément le capteur de pression (35) produit un signal de pression qui est lu par une unité de commande (43) pour commander une seconde fonction, dans lequel la première fonction est en particulier une fonction mécanique,
**caractérisé en ce que** ledit capteur de pression (35) est inclus dans la surface de préhension (68).

2. Dispositif médical (10) selon la revendication 1, comprenant en outre une paire de mâchoires (34, 36) avec une première mâchoire (34) et une seconde mâchoire (36), la seconde mâchoire (36) étant positionnée de manière adjacente à la première mâchoire (34), la paire de mâchoires (34, 36) étant mobile entre une position ouverte et une position fermée, dans lequel la fonction mécanique est une ouverture ou une fermeture de la paire de mâchoires (34, 36).

3. Dispositif médical (10) selon la revendication 2, dans lequel l'entrée fournie à la fonction mécanique est l'une des suivantes :
une entrée mécanique à un mécanisme qui déplace un tube (48) sur la paire de mâchoires (34, 36) pour fermer la première mâchoire (34) et la seconde mâchoire (36) ensemble de sorte qu'elles saisissent un tissu, et
une entrée mécanique à un mécanisme qui déplace la paire de mâchoires (34, 36) au-delà de l'extrémité du tube (48) pour ouvrir la première mâchoire (34) et la seconde mâchoire (36).

4. Dispositif médical (10) selon la revendication 2, dans lequel la première mâchoire (34) inclut une première électrode (146) et la seconde mâchoire (36) inclut une seconde électrode (152), la première électrode (146) et la seconde électrode (152) étant configurées pour délivrer de l'énergie au tissu.

5. Dispositif médical (10) selon la revendication 4, dans lequel la seconde fonction est une fonction électrique qui fournit de l'énergie à la paire d'électrodes (146, 152).

6. Dispositif médical (10) selon la revendication 5, dans lequel le signal de pression détermine la quantité d'énergie fournie à la paire d'électrodes (146, 152) et dans lequel un signal de pression augmentée est lié à une fonction continue.

7. Dispositif médical (10) selon la revendication 5, dans lequel le signal de pression détermine la quantité d'énergie fournie à la paire d'électrodes (146, 152) et dans lequel un signal de pression augmentée est lié de manière discrète d'une première puissance de sortie à une seconde puissance de sortie.

8. Dispositif médical (10) selon l'une quelconque des revendications précédentes, dans lequel un mouvement du levier (32) déclenche un interrupteur électrique pour fournir une entrée électrique.

9. Dispositif médical (10) selon la revendication 8, dans lequel l'entrée électrique active un sous-système électrique qui est en particulier un témoin lumineux ou un moteur de débrideur.

10. Dispositif médical (10) selon l'une quelconque des revendications précédentes, dans lequel l'unité de commande (43) active la seconde fonction lorsque le signal de pression est supérieur à une valeur de pression seuil.

11. Dispositif médical (10) selon la revendication 10, dans lequel la valeur de pression seuil est liée à une valeur seuil qui est supérieure à une force nécessaire pour déplacer le levier (32) sur toute sa plage de mouvement.

12. Dispositif médical (10) selon l'une quelconque des revendications précédentes, dans lequel le capteur de pression (35) est une résistance sensible à la force dont la résistance électrique diminue lorsque la force appliquée au capteur de pression (35) augmente.

13. Dispositif médical (10) selon la revendication 1, comprenant en outre :
un élément tubulaire allongé (12) avec une extrémité proximale (14) et une extrémité distale (16), l'extrémité proximale (14) étant reliée à la pièce à main (22) ;
une paire de mâchoires (34, 36) positionnées à l'extrémité distale (14) de l'élément tubulaire allongé (12), la paire de mâchoires (34, 36) incluant une première mâchoire (34) avec une première électrode (146) et une seconde mâchoire (36) avec une seconde électrode (152), la seconde mâchoire (36) étant positionnée de manière adjacente à la première mâchoire (34), la paire de mâchoires (34, 36) étant mobile entre une position ouverte et une position fermée, la première électrode (146) et la seconde électrode (152) étant configurées pour délivrer de l'énergie au tissu ;
dans lequel la première fonction est d'ouvrir ou de fermer la paire de mâchoires (34, 36) et la seconde fonction est une quantité d'énergie fournie à la première électrode (146) et à la seconde électrode (152), dans lequel la quantité d'énergie est en particulier suffisante pour coaguler le tissu.

14. Dispositif médical (10) selon la revendication 13, dans lequel l'unité de commande (43) fournit de l'énergie à la paire de mâchoires (34, 36) lorsque le signal de pression est supérieur à une valeur de pression seuil.
